# EUROPEAN PATENT SPECIFICATION

(11) **EP 2 437 689 B1**
(45) Date of publication and mention of the grant of the patent: **02.08.2023**
(21) Application number: 10784170.2
(22) Date of filing: 04.06.2010
(51) Int. Cl.: A61F 2/24

(54) **FLEXIBLE COMMISSURE STRUCTURE FOR ATTACHING VALVE BIOPROSTHESIS**
FLEXIBLE NAHTSTRUKTUR ZUR BEFESTIGUNG VON KLAPPENBIOPROTHESEN
STRUCTURE A COMMISSURE FLEXIBLE SERVANT A FIXER UNE BIOPROTHESE DE VALVULE

(30) Priority: 05.06.2009 US 184519 P
(43) Date of publication of application: 11.04.2012
(73) Proprietor: Medtronic ATS Medical Inc., Plymouth, Minnesota 55447 (US)
(72) Inventor: MALEWICZ, Andrzej M., Minneapolis Minnesota 55409 (US); WESTON, Matthew W., Little Canada Minnesota 55109 (US)
(74) Representative: Zimmermann & Partner Patentanwälte mbB
(86) International application number: PCT/US2010/037445
(87) International publication number: WO 2010/141847

(56) References cited:
- EP-A1- 1 690 515
- WO-A1-03/094797
- WO-A1-2005/046528
- WO-A1-2009/045338
- WO-A1-2009/045338
- WO-A1-2010/098857
- US-A1- 2005 075 584
- US-A1- 2006 178 740

## Description

### BACKGROUND OF THE INVENTION

### Field of the Invention.

The present invention relates to a bioprosthetic heart valve supported by a tubular, expandable anchoring structure having commissure posts of improved bending flexibility.

### Description of the Related Art.

Prosthetic heart valves may be used to replace diseased natural heart valves in human patients. Minimally invasive bioprosthetic heart valves typically include a proximal end or inflow ring and a distal end or outflow ring. At least two, but typically three, support structures extend from and connect the inflow ring to the outflow ring. These support structures are commonly referred to as commissure posts. In conventional devices these posts are rigid to provide support to the heart valve, but are also somewhat longitudinally flexible to allow them to be manipulated during implantation. The commissure posts define the juncture between adjacent tissue or synthetic leaflets otherwise secured thereto.

The body of the heart valve typically includes a plurality of multiple leaflets of valve tissue joined by seams with each seam formed by the junction of two leaflets. The inlet comprises an inflow annulus, preferably with either a scalloped or straight edge. The inflow ring may further optionally include a reinforcement structure, such as fabric, that can be stitched to it. Both the inflow and outflow rings are typically formed with an undulating or sinusoidal configuration, which are connected by the rigid commissure posts. The anchoring structure for a minimally invasive heart valve is typically made from a wire frame of metal that exhibits a high modulus of elasticity and that is biocompatible, such as Nitinol, as such materials exhibiting superior radial compressibility allow the anchoring structure to self-expand upon the release of the radially compressive forces. Optional, integrally formed commissural tabs attach the tissue segments to the commissural posts of the stent. Alternatively, the tissue heart valve may simply be attached to the commissural posts along the seam line or at a portion of the outflow end. During delivery and deployment, the stented heart valve resides in a radially-compressed or folded configuration in a delivery tool or catheter.

One problem associated with the delivery and deployment of a conventional stented bioprosthetic heart valves with rigid commissural posts is that binding, overlapping or interference can occur between adjacent heart valve segments. For example, the stented heart valve is typically placed in a delivery mechanism. As the delivery mechanism is threaded through the vasculature it bends as it follows the artery. In addition, upon deployment the delivery system containing the stented heart valve may curve slightly. In both of these cases, the inside radius of the bend causes wire segments of the anchoring structure to move toward each other causing overlapping. Moreover, on the outside radius of the bend, the wire segments may move away from each other, leaving large gaps that prevent the inflow ring from effectively seating the valve in the annulus. This can lead to improper annulus support, trauma, flow disturbance, kinking, paravalvular leakage, and interference with coronary ostia.

Still a further problem associated with minimally invasive bioprosthetic heart valves is the failure of the commissure posts to accommodate a curved aortic profile. This in turn leads to leaking, improper support of the annulus and potential trauma to the aortic wall.

Yet another problem is that the rigid commissure posts in conventional designs fail to allow the inflow and outflow rings to fully radially expand leading to leaking and improper seating of the valve in the annulus.

The present invention is directed to solving, or at least reducing, some or all of the aforementioned problems.

WO 2009/045338 A1, for example, relates to prosthetic heart valves, and more particularly to prosthetic heart valves that can be collapsed to a relatively small size for delivery into a patient and then re-expanded to full operating size at the final implant site in the patient.

EP1690515 A1 relates to a cardiac-valve prosthesis, which can be used, for example, as valve for percutaneous implantation, and comprises an armature for anchorage of the valve prosthesis in the implantation site, and wherein the armature comprises: two annular parts connected by anchorage formations, which extend as connection of the annular parts , with the capacity of projecting radially with respect to the prosthesis; and supporting formations for the leaflets, said formations being carried by at least one of the annular parts.

### SUMMARY OF THE INVENTION

The present invention is as set forth in the appended claims and advantageously provides a stented heart valve that includes a commissure structure which has a bending flexibility that is greater than those of conventional structures and avoids the problems associated with conventional stented bioprosthetic valves and also exhibits improved overall flexibility.

An exemplary embodiment of the invention provides a stented bioprosthetic heart valve that includes a plurality of commissural posts formed from a single wire having a sinusoidal wave pattern cut along a longitudinal axis thereof.

Another exemplary embodiment of the invention provides a minimally invasive bioprosthetic heart valve that includes a plurality of commissural posts formed from multiple wires each having a sinusoidal wave pattern cut along a longitudinal axis thereof.

Yet another exemplary embodiment of the invention provides a stented bioprosthetic heart valve that includes commissural posts formed by a single or double wire each including a generally sinusoidal wave pattern cut along a longitudinal length thereof wherein the bending flexibility of the commissure post increases as the frequency of the generally sinusoidal wave increases.

In yet another exemplary embodiment of the invention, a stented bioprosthetic heart valve includes commissural posts formed by a single or double wire each including a generally sinusoidal wave pattern cut along a longitudinal length thereof wherein the bending flexibility of the commissure post increases as the amplitude or wave height of the generally sinusoidal waves increases.

In yet another exemplary embodiment of the invention, a stented bioprosthetic heart valve includes commissural posts formed by a single or double wire each including a generally sinusoidal wave pattern cut along a longitudinal length thereof wherein the bending flexibility of the commissure post increases as the frequency and amplitude of the generally sinusoidal waves increases.

In yet another exemplary embodiment of the invention, a stented bioprosthetic heart valve includes commissural posts formed by a single or double wire each including a generally sinusoidal wave pattern cut along a longitudinal length thereof wherein the bending flexibility of the commissure post increases as the wavelength, or peak to peak distance, decreases.

In yet another exemplary embodiment of the invention, the flexible commissure posts have a bending flexibility along a plane defined by a surface containing all straight lines connecting any point on the central longitudinal axis of the cylindrical anchoring structure and any point on the longitudinal axis of the flexible commisure posts.

In yet another exemplary embodiment of the invention, a stented bioprosthetic heart valve includes commissural posts that are especially designed for replacement of aortic and pulmonary valves.

In another exemplary embodiment of the invention a valve assembly is provided, comprising a valve and anchoring structure with flexible commissure posts, in which the valve comprises a body having a proximal end and a distal end, an inlet at the proximal end, and an outlet at the distal end. The inlet comprises an inflow annulus, preferably with either a scalloped or straight edge. The outlet comprises a plurality of tabs that are supported by the commissural posts anchoring structure at the outlet end. In exemplary embodiments of the invention, the plurality of tabs is spaced evenly around the circumference of the valve.

It should be noted that for the purposes of this invention, the phrase "generally sinusoidal" is intended to include waves characterized by sine and cosine functions as well as waves which are not rigorously characterized by those functions, but nevertheless resemble such waves. In a more general way, such waves include those which are characterized as having one or more peaks and troughs. As an example, a wave whose peaks and troughs are U-shaped or bulbous is intended to be included. Also intended to be included, without limiting the definition, are waves which are more triangular in shape such as a saw-tooth wave or waves whose peaks and troughs are rectangular.

The flexible commissure post in accordance with the invention ensures complete or full radial expansion and deployment of the inflow and outflow rings thus providing for precise placement of the heart valve and a more open path for blood flow.

### BRIEF DESCRIPTION OF THE DRAWINGS

FIG. 1 shows an exemplary valve during normal operation. FIG. 1A shows the valve in the open position during peak flow. FIG. 1B shows the valve in closed position to prevent backflow of the fluid across the valve.
FIG. 2 is an exemplary bioprosthetic tissue valve for use with the present invention.
FIG. 3A depicts an exemplary embodiment of a tubular anchoring device including flexible commissure posts in accordance with one aspect of the present invention cut along line A-A and laid flat.
FIG. 3B depicts a variation of the tubular anchoring device with flexible commissure posts illustrated in FIG. 3A.
FIG. 4A depicts a further embodiment of one aspect of a tubular anchoring device with flexible commissure posts in accordance with the present invention cut along line B-B and laid flat.
FIG. 4B depicts a variation of the tubular anchoring device with flexible commissure posts illustrated in FIG. 4A.
FIG. 5A depicts an exemplary embodiment of the bioprosthetic heart valve of FIG. 2 supported by the tubular anchoring structure with flexible commisure posts of the present invention.
FIG. 5B is a top view of the cylindrical anchoring structure showing the longitudinal axis L' through the center of the cylindrical anchoring structure.
FIG. 6 depicts the exemplary bioprosthetic heart valve supported by the tubular anchoring structure with flexible commisure posts of the present invention positioned within an aorta.

### DETAILED DESCRIPTION OF THE INVENTION

While this invention may be embodied in many different forms, there are described in detail herein various embodiments of the invention. This description is an exemplification of the principles of the invention and is not intended to limit the invention to the particular embodiments illustrated.

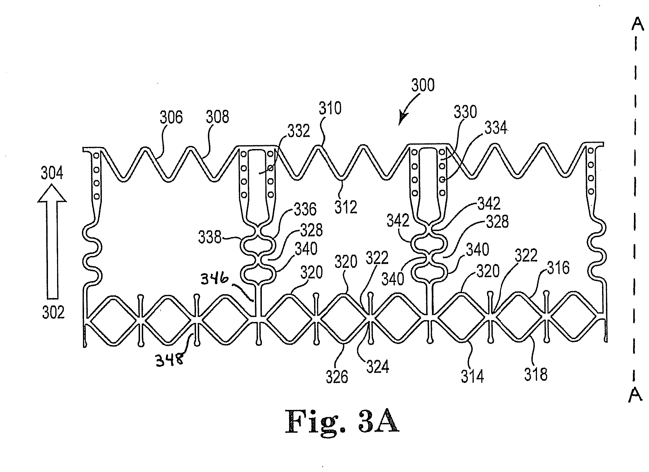

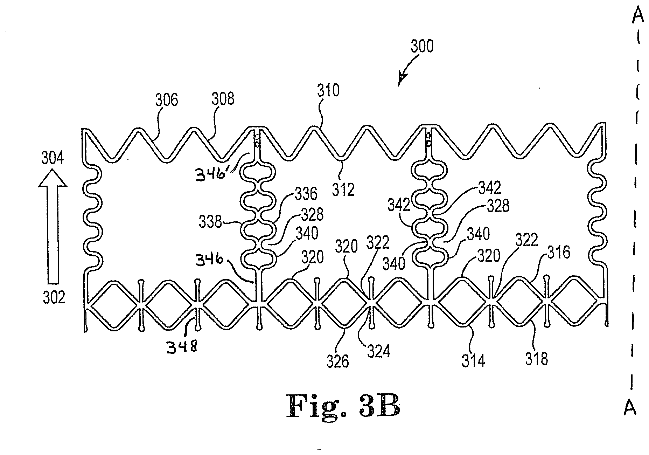

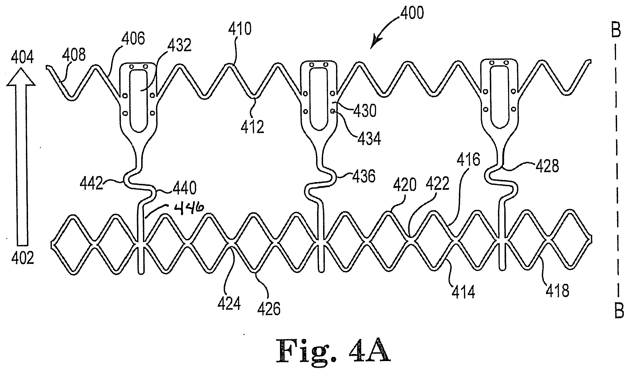

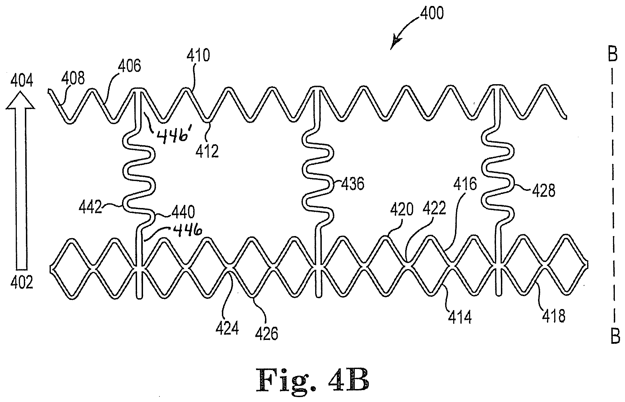

When referring to the terms "peak" and "trough" as they relate to the commissure posts these terms are defined with respect to the left and right sides of the figures, respectively. As seen in the figures, each of the commissure posts has a paddle end and a proximal/straight end. With regard to the commissure posts, peaks are the first and subsequent sinusoidal wave commencing at the inflow end and are concave relative to the right side of the respective figure and convex relative to the left side of the respective figure. Troughs as they pertain to the commissure post, on the other hand, are convex relative to the right side of the figure and concave relative to the left side of the figure. As may be appreciated by those of ordinary skill in the art, the commissure post may have a sinusoidal wave formed from one wire, as depicted in FIGS. 4A and 4B or may have sinusoidal waves formed from multiple wires, as depicted in FIGS. 3A and 3B.

In addition given constant wave amplitude, as the frequency of the sinusoidal waves increases, the bending flexibility of the commissure posts increases. Further, given constant wave frequency, as the amplitude of the waves increases the bending flexibility of the commissure posts increases. Moreover, increasing wave amplitude and wave frequency result in an increase in commissure post bending flexibility. In addition, as the peak to peak distance, or wavelength, between each wave decreases, the bending flexibility of the commissure post increases.

As seen in the figures, the tubular or generally cylindrical anchoring structure includes an inflow end with an inflow ring and an outflow end with an outflow ring. With respect to the inflow and outflow rings peaks are concave relative to the proximal end of the anchoring structure and convex relative to the distal end of the anchoring structure. Troughs, on the other hand, are convex relative to the proximal end of the anchoring structure and concave relative to the distal end of the anchoring structure.

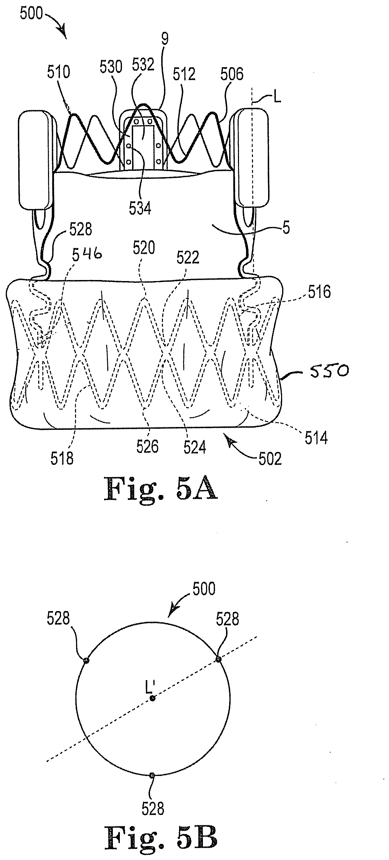

In addition, by "flexible" commissure posts and enhanced or greater "flexibility" we mean the bending capacity or bending flexibility of the commissure posts of the stented heart valve in or along a plane defined by the surface containing all the straight lines that connect any point on the longitudinal axis running through the center of the cylindrical anchoring structure (as seen in FIG. 5B) and any point on the longitudinal axis of the commissure post (as seen in FIG. 5A). The bending flexibility of the commissure posts in accordance with the invention is 10% or more than conventional rigid posts, which tend to flex only along the longitudinal axis of the post.

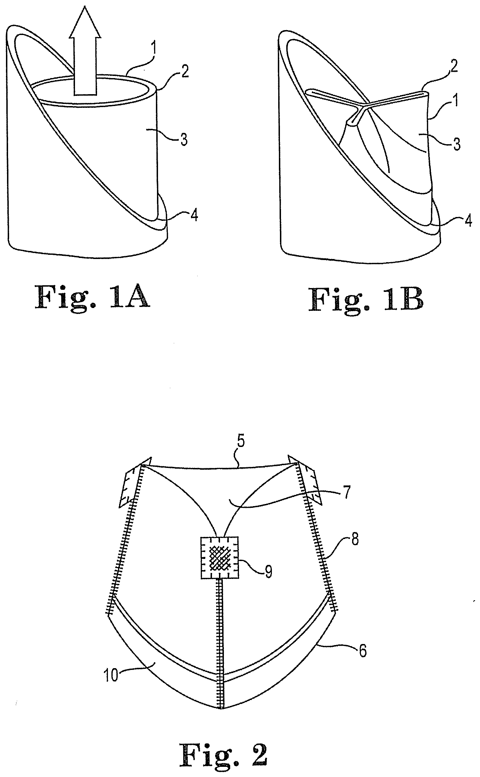

Turning now to the figures, the invention relates to valve replacement systems including a tubular anchoring structure that has flexible commissure posts. As illustrated in FIG. 1, a valve (1) comprises a distal or outflow end (2), leaflets (3) and a proximal or inflow end (4). A typical valve functions similar to a collapsible tube in that it opens widely during systole or in response to muscular contraction, to enable unobstructed forward flow across the valvular orifice (FIG. 1A). In contrast, at the end of systole or contraction, as illustrated in FIG. 1B, as forward flow decelerates, the walls of the tube are forced centrally between the sites of attachment to the vessel wall and the valve closes completely.

One embodiment of a bioprosthetic valve 5 for use with the system of the present invention is illustrated in FIG. 2 and is comprised of a body having a proximal end or inflow portion 6 and a distal end or outflow portion 7. The body is comprised of multiple leaflets of valve tissue joined by seams 8, wherein each seam is formed by a junction of two leaflets. An optional commissural tab region 9, which may be integrally formed from adjacent leaflets, extends from each seam at the distal end of the valve body. The proximal end 6 has a peripheral edge that can be scalloped or straight. The proximal end or inflow 6 of the valve can further comprise an optional reinforcement structure 10 that can be stitched to it. In preferred embodiments of the invention, the inflow edge of the valve is scalloped. The novel design for a bioprosthetic heart valve system supported by a tubular, expandable anchoring structure having commissure posts of improved bending flexibility is not limited, however, to the specific valve illustrated in FIG. 2.

Turning now to FIG. 3A one exemplary embodiment of a tubular, expandable anchoring structure 300 including commissure posts of improved bending flexibility is shown. Blood inflow is depicted at 302 and outflow is depicted at 304. The anchoring structure is generally cylindrical in shape but for purposes of illustration is depicted as being cut along line A-A and laid flat. The anchoring structure 300 includes an outflow ring 306 having a single sinusoidal-shaped wire 308 including peaks 310 and troughs 312. Anchoring structure 300 also includes inflow ring 314 having two sinusoidal-shaped wires designated as distal inflow wire ring 316 and proximal inflow wire ring 318. Distal inflow wire ring 316 includes peaks 320 and troughs 322. Proximal inflow wire ring 318 includes peaks 324 and troughs 326. As can be seen from FIG. 3A the troughs 322 of distal inflow wire ring 316 are joined to the peaks 324 of proximal inflow wire ring 318. Those of ordinary skill in the art will appreciate that although two inflow wires forming inflow ring 314 are depicted, a single wire, triple wires or any other configuration may also be used. At the outflow end, flexible commissure posts 328 include paddle portion 330 that couples the commissure posts 328 to the outflow ring 306. Paddle portion 330 with axial slot 332 extends along the longitudinal axis of commissure post 330. Suturing holes 334 are positioned on the outer periphery of paddle 330 and are used to suture a bioprosthetic heart valve, such as shown in FIG. 2, to the anchoring structure 300. Commissure post 328 includes first and second commissure post wires 336, 338 each with peaks 340 and troughs 342 that converge to a single wire 346 at the inflow end that couples the flexible commisure posts to the inflow ring 314. As can be seen from FIG. 3A the first commissure post wire 336 is joined at trough 342 to a peak 340 of the second commissure wire 338. The number of commissure posts 328 in this embodiment can range from two to four, depending on the number of leaflets present in the valve sinus. Thus, in one embodiment the anchoring structure comprises three support posts for a three-leaflet valve with a sinus that features three natural commissural posts. The commissure posts 328 of the anchoring structure 300 are configured to coincide with the natural commissural points of the sinus. The inflow ring 314 optionally includes finger-like elements 348 positioned between distal and proximal inflow wires and extend in an axial direction therefrom. Finger-like elements 348 are designed to lend additional support to fabric that may cover inflow rim 314 to anchor the fabric and permit tissue ingrowth. In addition, although only two waves are shown those of ordinary skill in the art will appreciate that the number of waves may be varied to achieve the desired bending flexibility.

FIG. 3B depicts a variation of the tubular, expandable anchoring structure 300 including commissure posts of improved bending flexibility of FIG. 3A. In FIG. 3B paddle portion 330 has been eliminated and a single wire 346' extends from the commissure post which couples the commissure post directly to the outflow ring. Single wire 346' may contain suturing holes to attach the tissue heart valve to the commisure posts. The anchoring structure 300 includes an outflow ring 306 having a single sinusoidal-shaped wire 308 including peaks 310 and troughs 312. Anchoring structure 300 also includes inflow ring 314 having two sinusoidal-shaped wires designated as distal inflow wire ring 316 and proximal inflow wire ring 318. Distal inflow wire ring 316 includes peaks 320 and troughs 322. Proximal inflow wire ring 318 includes peaks 324 and troughs 326. As can be seen from FIG. 3B the troughs 322 of distal inflow wire ring 316 are joined to the peaks 324 of proximal inflow wire ring 318. Those of ordinary skill in the art will appreciate that although two inflow wires forming inflow ring 314 are depicted, a single wire, triple wires or any other configuration may also be used. Commissure post 328 includes first and second commissure post wires 336, 338 each with peaks 340 and troughs 342. As can be seen from FIG. 3B the first commissure post wire 336 is joined at trough 342 to a peak 340 of the second commissure wire 338. The number of commissure posts 328 in this embodiment can range from two to four, depending on the number of leaflets present in the valve sinus. Thus, in one embodiment the anchoring structure comprises three support posts for a three-leaflet valve with a sinus that features three natural commissural posts. The commissure posts 328 of the anchoring structure 300 are configured to coincide with the natural commissural points of the sinus. In addition, while multiple waves are depicted the actual number of waves may vary depending on the desired bending flexibility.

Turning now to FIG. 4A another exemplary embodiment of a tubular, expandable anchoring structure 400 including commissure posts of improved bending flexibility is shown. Blood inflow is depicted at 402 and outflow is depicted at 404. The anchoring structure 400 includes an outflow ring 406 having a single sinusoidal-shaped wire 408 including peaks 410 and troughs 412. Anchoring structure 400 also includes inflow ring 414 having first and second sinusoidal-shaped wires designated as distal inflow wire ring 416 and proximal inflow wire ring 418. Distal inflow wire ring 416 includes peaks 420 and troughs 422. Proximal inflow wire ring 418 includes peaks 424 and troughs 426. As can be seen from FIG. 4A the troughs 422 of distal inflow wire ring 416 are joined to the peaks 424 of proximal inflow wire ring 418. One of ordinary skill in the art will appreciate that although two inflow wires forming inflow ring 414 are depicted, a single wire, triple wires or any other configuration may also be used. Flexible commissure posts 428 include paddle portion 430 with axial slot 432 at the outflow end and extending along the longitudinal axis of commissure post 430. Suturing holes 434 are positioned on the outer periphery of paddle 430 and are used to suture a bioprosthetic heart valve, such as shown in FIG. 2, to the anchoring structure 400. Commissure post 428 includes a single sinusoidal wave like wire 436 with peaks 440 and troughs 442 which couples to the inflow ring 414 by single wire 446. The number of commissure posts 428 in this embodiment can range from two to four, depending on the number of leaflets present in the valve sinus. Thus, in one embodiment the anchoring structure comprises three support posts for a three-leaflet valve with a sinus that features three natural commissural posts. The commissure posts 428 of the anchoring structure 400 are configured to coincide with the natural commissural posts of the sinus. In addition, although only two waves are shown those of ordinary skill in the art will appreciate that the number of waves may be varied to achieve the desired bending flexibility.

FIG. 4B depicts a variation of the tubular, expandable anchoring structure 400 including commissure posts of improved bending flexibility of FIG. 4A. In FIG. 4B paddle portion 430 has been eliminated and a single wire 446' extends from the commissure post which couples the commissure post directly to the outflow ring. Single wire 346' may contain suturing holes to attach the tissue heart valve to the commisure posts. The anchoring structure 400 includes an outflow ring 406 having a single sinusoidal-shaped wire 408 including peaks 410 and troughs 412. Anchoring structure 400 also includes inflow ring 414 having two sinusoidal-shaped wires designated as distal inflow wire ring 416 and proximal inflow wire ring 418. Distal inflow wire ring 416 includes peaks 420 and troughs 422. Proximal inflow wire ring 418 includes peaks 424 and troughs 426. As can be seen from FIG. 4B the troughs 422 of distal inflow wire ring 416 are joined to the peaks 424 of proximal inflow wire ring 418. Those of ordinary skill in the art will appreciate that although two inflow wires forming inflow ring 414 are depicted, a single wire, triple wires or any other configuration may also be used. Commissure post 428 includes a single commissure post wire 436 each with peaks 440 and troughs 442. The number of commissure posts 428 in this embodiment can range from two to four, depending on the number of leaflets present in the valve sinus. Thus, in one embodiment the anchoring structure comprises three support posts for a three-leaflet valve with a sinus that features three natural commissural posts. The commissure posts 428 of the anchoring structure 400 are configured to coincide with the natural commissural points of the sinus. In addition, while multiple waves are depicted the actual number of waves may vary depending on the desired bending flexibility.

FIG. 5 shows an exemplary bioprosthetic valve system including the exemplary anchoring structure with flexible commissure posts depicted in FIGS. 4A. Those of ordinary skill in the art will appreciate that the anchoring structure of FIG. 3A, 3B or 4B may also be used.

The anchoring structure 500 is adapted to support a valve such as that illustrated in FIG. 2. As seen in FIG. 5A, the anchoring structure 500 has a generally tubular or cylindrical configuration within which a bioprosthetic heart valve 5 is secured. The valve 5 is secured at its proximal (inflow) annulus by attachment to the inflow ring 514 of the anchoring structure 500 and at its distal end via the optional commissural tabs 9 that are threaded through the axially extending slots 532 of the paddle portion 530. Paddle portion 530 couples the flexible commissure posts 528 to the outflow ring 506 of the anchoring structure 500, whereas the proximal end of the commissure posts 528 are coupled to the inflow ring 514 of the anchoring structure 500 by single wire connector 546.

In FIG. 5A the outflow ring 506 of the anchoring structure 500 is depicted as comprising a single sinusoidal-shaped wire that extends between commisure posts 528 generally at or above the axially extending slots 532 that reside therein. The single wire of the outflow ring 506 is configured in an undulating or sinusoidal pattern forming peaks 510 and troughs 512. Anchoring structure 500 also includes inflow ring 514 having first and second sinusoidal-shaped wires designated as distal inflow wire ring 516 and proximal inflow wire ring 518. Distal inflow wire ring 516 includes peaks 520 and troughs 522. Proximal inflow wire ring 518 includes peaks 524 and troughs 526. As can be seen from FIG. 5A the troughs 522 of distal inflow wire ring 516 are joined to the peaks 524 of proximal inflow wire ring 518. This arrangement allows the distal inflow wire ring and proximal inflow wire ring to move together when the valve is in its radially compressed state prior to delivery thus preventing possible damage to the bioprosthetic heart valve.

Flexible commissure posts 528 include paddle portion 530 with axial slot 532 extending along the longitudinal axis (L) of commissure post 528. Suturing holes 534 are positioned on the outer periphery of paddle 530 and are used to suture a bioprosthetic heart valve, such as shown in FIG. 2, to the anchoring structure 500. Paddle portion 530 of commissure post 528 connects commissure post 528 to the single wire of inflow ring 506 at trough 512. The single wire connector 546 couples commissure post 528 to the inflow ring 514. Single wire connector 546 is designed to stabilize the anchoring structure and to prevent distortion of the valve during compression and expansion. The single wire connector 546 extends longitudinally in the axial direction of the cylindrical anchoring structure 500. Those of ordinary skill in the art will appreciate that both the inflow ring 514 and outflow ring 506 can be comprised of any number of wires without deviating from the spirit of the present invention.

Both the inflow 514 and outflow 506 rings of the anchoring structure 500 are formed with an sinusoidal wave configuration, although the inflow ring 514 may have a longer wavelength (circumferential dimension from peak to peak) and a lesser wave height (axial dimension from peak to peak) than the outflow ring 506. The wavelengths and wave heights of the inflow 514 and outflow 506 rings are selected to ensure uniform compression and expansion of the anchoring structure without distortion. The wavelength of the inflow rim 514 is further selected to support the geometry of the scalloped inflow annulus of a preferred valve of the present invention. Notably, as shown in FIG. 5, the sinusoidal wave pattern that forms the distal and proximal wires 516, 518 of inflow ring 514 is configured such that the single wire connector 546 is connected to the point at which peaks 524 and troughs 522 meet. Similarly, the undulating or sinusoidal wave pattern that forms the outflow ring 506 of the anchoring structure 500 is configured such that the paddle portion 530 of the commissure posts 528 is connected to the trough 512 of the single wire outflow ring 506. Locating the paddle portion of the commissure post 528 at the troughs 512 of the outflow ring 506 will prevent the longitudinal extension of outflow ring in the direction of the valve secured within the lumen of the anchoring structure upon compression of the valve assembly, thereby eliminating any contact between valve and anchoring structure. Thus, compression of the valve and anchoring structure 500 does not lead to distortion of or injury to the tissue valve.

FIG. 5 further shows that the commissure posts 528 include sinusoidal-shaped waves cut thereinto. An important function of the commissure posts 528 is the stabilization of the valve in general, and in particular the prevention of any longitudinal extension at points of valve attachment to preclude valve stretching or distortion upon compression of the device. Another important function of the sinusoidal-shaped commissure posts is to provide for axial and bending decoupling of the inflow and outflow rings to ensure complete circumferential expansion and deployment of the inflow and outflow rings during placement thus providing a more open path for blood flow to the coronary ostia and through the coronary artery. As will be appreciated by those of skill in the art, the wavelengths, frequency and amplitude of the waves of the commissure posts in accordance with the present invention are selected to ensure uniform compression and expansion of the anchoring structure without distortion upon deployment and to ensure that the inflow ring expands to its full circumferential profile to allow for proper seating within the annulus.

The number of support posts 528 in this preferred embodiment can range from two to four, depending on the number of commissural posts present in the valve sinus. Thus, in a preferred embodiment, the anchoring structure comprises three support posts for a three-leaflet valve with a sinus that features three natural commissural posts. The support posts 528 of the anchoring structure 500 are configured to coincide with the natural commissural posts of the sinus.

As can be seen from FIG. 5, inflow ring 514 may be covered with a cloth or fabric material 550. The fabric 550 may comprise any suitable material including, but not limited to, woven polyester, polyester velour, polyethylene terepthalate, polytetrafluoroethylene (PTFE), or other biocompatible material. Fabric 550 permits for tissue ingrowth over time to firmly position the minimally invasive bioprosthetic valve system in the annulus.

FIG. 5B depicts the longitudinal axis (L') running through the center of the cylindrical anchoring structure, which in turn defines the plane of commissural post bending flexibility as hereinbefore noted.

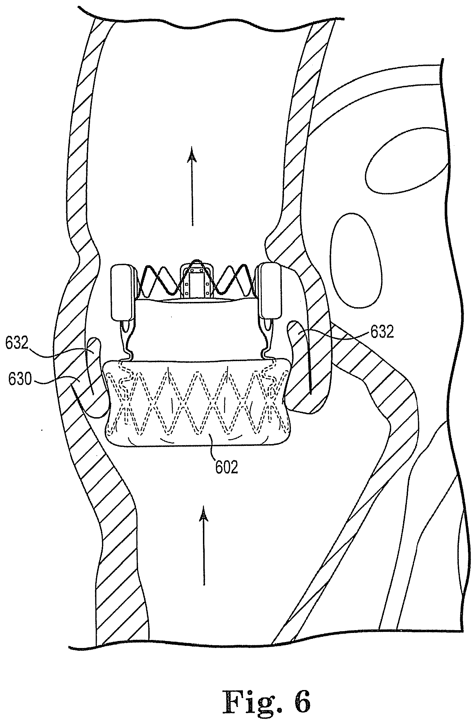

FIG. 6 shows a bioprosthetic valve system including the exemplary anchoring structure with flexible commissure posts of FIG. 4A fully seated within an aorta after deployment. Those of ordinary skill in the art will appreciate that the anchoring structure of FIG. 3A, 3B or 4B and variations thereof may also be used. In the exemplary embodiment discussed above, a crimping tool may be used to crimp the exemplary heart valve supported by the anchoring structure with flexible commissure posts. The crimped valve is loaded into a delivery device, known to those of ordinary skill in the art, which is then used to deliver the crimped stented heart valve to an aortic annulus. The crimped stented heart valve may be delivered surgically or transapically. In surgical placement, the patient may be put on bypass and the aorta at least partially transected. The surgeon then positions the delivery device within the aortic annulus 630, radially compressing the native leaflets 632 as the crimped heart valve expands, such that the exposed inflow end 602 is substantially aligned with the inflow annulus of the native valve. As will be appreciated by those of ordinary skill in the art, warm bodily fluids may cause the exposed portion of the stented heart valve 600, i.e. the inflow end 602, to start to expand to the "remembered" shape as further illustrated in FIG. 6. Alternatively or in addition, the surgeon may apply a warm solution to the implantation site to promote re-expansion of the stented heart valve 600, such as a warm saline solution.

As the stented heart valve 600 starts to expand the delivery device 630 may be retracted to expose an additional length of the inflow end 602 of the stented heart valve 600 until the stented heart valve 600 completely expands in the aortic annulus 630 where it friction fits and seals into place. The bending flexibility of the commissure posts ensures that binding, overlapping and/or interference between adjacent segments of the anchoring structure with heart valve is minimized and/or eliminated. In addition, the enhanced bending flexibility of the commissure posts ensures that the heart valve is properly seated in the aortic annulus.

Although the present invention has been described with reference to preferred embodiments, workers skilled in the art will recognize that changes may be made in form and detail without departing from the scope of the appended claims.

## Claims

1. A structure for supporting a bioprosthetic heart valve comprising:
a cylindrical anchoring structure (300) defining a central longitudinal axis, the cylindrical anchoring structure comprising:
an inflow ring (314) including at least one sinusoidal-shaped wire (316) having a plurality of peaks (320) and troughs (322);
an outflow ring (306) including at least one sinusoidal-shaped wire (308) having a plurality of peaks (310) and troughs (312), and
at least one flexible commissure post (328) having a longitudinal axis, the flexible commissure post (328) connecting the inflow ring (314) and the outflow ring (306),
wherein the flexible commissure post (328) has a bending flexibility along a plane defined by a surface containing all straight lines connecting any point on the central longitudinal axis of the cylindrical anchoring structure and any point on the longitudinal axis of the flexible commissure post (328), wherein the at least one flexible commissure post (328) includes a paddle portion (330) operably connected to the outflow ring (306), the paddle portion (330) including an axial slot (332) extending along the longitudinal axis thereof.

2. The anchoring structure of claim 1 wherein the at least one flexible commissure post (328) includes a single wire having at least one peak and one trough.

3. The anchoring structure of claim 2 wherein the single wire has a plurality of peaks and troughs forming a plurality of generally sinusoidal waves.

4. The anchoring structure of claim 3 wherein the frequency and/or the wavelength of the sinusoidal waves varies along the longitudinal axis of the flexible commissure post (328).

5. The anchoring structure of claim 3 wherein the wavelength of the sinusoidal waves is substantially constant along the longitudinal axis of the flexible commissure post (328).

6. The anchoring structure of claim 3 wherein the bending flexibility of the flexible commissure post (328) increases as the frequency of the sinusoidal waves increases and/or the bending flexibility of the flexible commissure post (328) increases as the wave height of the sinusoidal waves increases and/or the bending flexibility of the flexible commissure post (328) increases as the peak-to-peak distance between adjacent waves decreases.

7. The anchoring structure of claim 2 wherein the inflow ring (314) includes a first sinusoidal-shaped wire (316) and a second sinusoidal-shaped wire (318), the first and second sinusoidal-shape wires (316, 318) each having a plurality of peaks (320) and troughs (322).

8. The anchoring structure of claim 7 wherein the peaks of the first sinusoidal-shaped wire (316) are joined to the troughs of the second sinusoidal- shaped wire (318).

9. The anchoring structure of claim 8 wherein the at least one flexible commissure post includes a vertical connection member, the vertical connection member joined to the inflow ring at the intersection between one of the peaks of the first sinusoidal-shaped wire and one of the troughs of the second sinusoidal-shaped wire.

10. The anchoring structure of claim 1 wherein the at least one flexible commissure post (328) includes first and second wires (336, 338), each of the wires including at least one peak (340) and one trough (342).

11. The anchoring structure of claim 10 wherein the at least one peak (340) of the first wire (336) abuts the at least one trough (342) of the second wire (338).

12. The anchoring structure of claim 11 wherein the first and second wires (336, 338) each include a plurality of peaks (340) and troughs (342) forming a plurality of generally sinusoidal waves.

13. The anchoring structure of claim 12 wherein the bending flexibility of the flexible commissure post (328) increases as the frequency of the sinusoidal waves of the first and second wires (336, 338) increases and/or the bending flexibility of the flexible commissure post (328) increases as the wave height of the sinusoidal waves of the first and second wires (336, 338) increases and/or the bending flexibility of the flexible commissure post (328) increases as the peak-to-peak distance between adjacent waves of the first and second wires (336, 338) decreases.

14. The anchoring structure of claim 1 wherein the bending flexibility of the commissure post (328) is greater than 10% of a rigid or semi-rigid commissure post.

15. The anchoring structure of claim 1 wherein the paddle portion (330) of the at least one flexible commissure post (328) is connected to one of the troughs (312) or to one of the peaks (312) of the outflow ring (306).

16. The anchoring structure of claim 1 further comprising a bioprosthetic heart valve (5) disposed within the cylindrical anchoring structure, the bioprosthetic heart valve including a plurality of leaflets defining a valve body having an inflow end and an outflow end.

17. The anchoring structure of claim 16 wherein adjacent leaflets are joined together by a seam (8) at the junction of the leaflets, and wherein a commissural tab (9) extends from each of the seams (8) near the outflow end (7) of the valve body.

18. The anchoring structure of claim 17 wherein the cylindrical anchoring structure includes a plurality of flexible commissure posts (328), and wherein the bioprosthetic heart valve (5) is secured to the cylindrical anchoring structure by threading the commissural tabs (9) through the axial slots (332) in the paddle portions (330) of the flexible commissure posts (328).

19. The anchoring structure of claim 18 wherein the commissural tabs (9) are formed integral with the leaflets.

20. The anchoring structure of claim 18 further comprising at least one suture hole (334) formed in each of the paddle portions (330) for suturing the commissural tabs (9) to the paddle portions (330).

21. The anchoring structure of claim 18 further comprising a biocompatible cloth covering (550) positioned around at least a portion of the inflow ring (514).

## Patentansprüche

1. Struktur zum Stützen einer bioprothetischen Herzklappe, umfassend:
eine zylindrische Verankerungsstruktur (300), die eine zentrale Längsachse definiert, die zylindrische Verankerungsstruktur umfassend:
einen Einströmring (314), der mindestens einen sinusförmigen Draht (316) beinhaltet, der eine Vielzahl von Spitzen (320) und Vertiefungen (322) aufweist;
einen Ausströmring (306), der mindestens einen sinusförmigen Draht (308) beinhaltet, der eine Vielzahl von Spitzen (310) und Vertiefungen (312) aufweist, und
mindestens einen flexiblen Kommissurpfosten (328), der eine Längsachse aufweist, wobei der flexible Kommissurpfosten (328) den Einströmring (314) mit dem Ausströmring (306) verbindet, wobei der flexible Kommissurpfosten (328) eine Biegeflexibilität entlang einer Ebene aufweist, die durch eine Oberfläche definiert ist, die alle geraden Linien enthält, die einen beliebigen Punkt auf der zentralen Längsachse der zylindrischen Verankerungsstruktur und einen beliebigen Punkt auf der Längsachse des flexiblen Kommissurpfostens (328) verbinden, wobei der mindestens eine flexible Kommissurpfosten (328) einen Paddelabschnitt (330) beinhaltet, der mit dem Ausströmring (306) wirkverbunden ist, wobei der Paddelabschnitt (330) einen axialen Schlitz (332) beinhaltet, der sich entlang der Längsachse davon erstreckt.

2. Verankerungsstruktur nach Anspruch 1, wobei der mindestens eine flexible Kommissurpfosten (328) einen einzelnen Draht beinhaltet, der mindestens eine Spitze und eine Vertiefung aufweist.

3. Verankerungsstruktur nach Anspruch 2, wobei der einzelne Draht eine Vielzahl von Spitzen und Vertiefungen aufweist, die eine Vielzahl von im Allgemeinen sinusförmigen Wellen ausbildet.

4. Verankerungsstruktur nach Anspruch 3, wobei die Frequenz und/oder die Wellenlänge der sinusförmigen Wellen entlang der Längsachse des flexiblen Kommissurpfostens (328) variiert.

5. Verankerungsstruktur nach Anspruch 3, wobei die Wellenlänge der sinusförmigen Wellen entlang der Längsachse des flexiblen Kommissurpfostens (328) im Wesentlichen konstant ist.

6. Verankerungsstruktur nach Anspruch 3, wobei die Biegeflexibilität des flexiblen Kommissurpfostens (328) zunimmt, wenn die Frequenz der sinusförmigen Wellen zunimmt und/oder die Biegeflexibilität des flexiblen Kommissurpfostens (328) zunimmt, wenn die Wellenhöhe der sinusförmigen Wellen zunimmt und/oder die Biegeflexibilität des flexiblen Kommissurpfostens (328) zunimmt, wenn der Spitze-zu-Spitze-Abstand zwischen angrenzenden Wellen abnimmt.

7. Verankerungsstruktur nach Anspruch 2 wobei der Einströmring (314) einen ersten sinusförmigen Draht (316) und einen zweiten sinusförmigen Draht (318) beinhaltet, wobei der erste und der zweite sinusförmige Draht (316, 318) jeweils eine Vielzahl von Spitzen (320) und Vertiefungen (322) aufweisen.

8. Verankerungsstruktur nach Anspruch 7, wobei die Spitzen des ersten sinusförmigen Drahts (316) mit den Vertiefungen des zweiten sinusförmigen Drahts (318) zusammengefügt werden.

9. Verankerungsstruktur nach Anspruch 8, wobei der mindestens eine flexible Kommissurpfosten ein vertikales Verbindungselement beinhaltet, wobei das vertikale Verbindungselement mit dem Einströmring an der Kreuzung zwischen einer der Spitzen des ersten sinusförmigen Drahts und einer der Vertiefungen des zweiten sinusförmigen Drahts zusammengefügt ist.

10. Verankerungsstruktur nach Anspruch 1, wobei der mindestens eine flexible Kommissurpfosten (328) einen ersten und einen zweiten Draht (336, 338) beinhaltet, wobei jeder der Drähte mindestens eine Spitze (340) und eine Vertiefung (342) beinhaltet.

11. Verankerungsstruktur nach Anspruch 10, wobei die mindestens eine Spitze (340) des ersten Drahts (336) an der mindestens einen Vertiefung (342) des zweiten Drahts (338) anliegt.

12. Verankerungsstruktur nach Anspruch 11, wobei der erste und der zweite Draht (336, 338) jeweils eine Vielzahl von Spitzen (340) und Vertiefungen (342) beinhalten, die eine Vielzahl im Allgemeinen sinusförmiger Wellen ausbildet.

13. Verankerungsstruktur nach Anspruch 12, wobei die Biegeflexibilität des flexiblen Kommissurpfostens (328) zunimmt, wenn die Frequenz der sinusförmigen Wellen des ersten und des zweiten Drahts (336, 338) zunimmt und/oder die Biegeflexibilität des flexiblen Kommissurpfostens (328) zunimmt, wenn die Wellenhöhe der sinusförmigen Wellen des ersten und des zweiten Drahts (336, 338) zunimmt und/oder die Biegeflexibilität des flexiblen Kommissurpfostens (328) zunimmt, wenn der Spitze-zu-Spitze-Abstand zwischen angrenzenden Wellen des ersten und zweiten Drahts (336, 338) abnimmt.

14. Verankerungsstruktur nach Anspruch 1, wobei die Biegeflexibilität des Kommissurpfostens (328) über 10 % eines starren oder halbstarren Kommissurpfostens ist.

15. Verankerungsstruktur nach Anspruch 1, wobei der Paddelabschnitt (330) des mindestens einen flexiblen Kommissurpfostens (328) mit einer der Vertiefungen (312) oder mit einer der Spitzen (312) des Ausströmrings (306) verbunden ist.

16. Verankerungsstruktur nach Anspruch 1, ferner umfassend eine bioprothetische Herzklappe (5), die innerhalb der zylindrischen Verankerungsstruktur angeordnet ist, wobei die bioprothetische Herzklappe eine Vielzahl von Segeln beinhaltet, die einen Klappenkörper definiert, der ein Einströmende und ein Ausströmende aufweist.

17. Verankerungsstruktur nach Anspruch 16, wobei angrenzende Segel durch eine Naht (8) an der Verbindungsstelle der Segel miteinander verbunden sind, und wobei sich eine Kommissurlasche (9) von jeder der Nähte (8) nahe dem Ausströmende (7) des Klappenkörpers erstreckt.

18. Verankerungsstruktur nach Anspruch 17, wobei die zylindrische Verankerungsstruktur eine Vielzahl von flexiblen Kommissurpfosten (328) beinhaltet, und wobei die bioprothetische Herzklappe (5) an der zylindrischen Verankerungsstruktur durch Winden der Kommissurlaschen (9) durch die axialen Schlitze (332) hindurch in die Paddelabschnitte (330) der flexiblen Kommissurpfosten (328) befestigt ist.

19. Verankerungsstruktur nach Anspruch 18, wobei die Kommissurlaschen (9) mit den Segeln einstückig ausgebildet sind.

20. Verankerungsstruktur nach Anspruch 18, ferner umfassend mindestens ein Fadenloch (334), das in jedem der Paddelabschnitte (330) zum Vernähen der Kommissurlaschen (9) an den Paddelabschnitten (330) ausgebildet ist.

21. Verankerungsstruktur nach Anspruch 18, ferner umfassend eine biokompatible Stoffabdeckung (550), die um mindestens einen Abschnitt des Einströmrings (514) herum positioniert ist.

## Revendications

1. Structure permettant de supporter une valvule cardiaque bioprothétique comprenant :
une structure d'ancrage cylindrique (300) définissant un axe longitudinal central, la structure d'ancrage cylindrique comprenant :
un anneau de flux entrant (314) comportant au moins un fil de forme sinusoïdale (316) ayant une pluralité de pics (320) et de creux (322) ;
un anneau de flux sortant (306) comportant au moins un fil de forme sinusoïdale (308) ayant une pluralité de pics (310) et de creux (312), et
au moins un montant commissural flexible (328) ayant un axe longitudinal, le montant commissural flexible (328) reliant l'anneau de flux entrant (314) et l'anneau de flux sortant (306), dans lequel le montant commissural flexible (328) a une flexibilité de flexion le long d'un plan défini par une surface contenant toutes les lignes droites reliant un quelconque point sur l'axe longitudinal central de la structure d'ancrage cylindrique et un quelconque point sur l'axe longitudinal du montant commissural flexible (328), dans lequel l'au moins un montant commissural flexible (328) comporte une partie palette (330) reliée fonctionnellement à l'anneau de flux sortant (306), la partie palette (330) comportant une fente axiale (332) s'étendant le long de l'axe longitudinal de celle-ci.

2. Structure d'ancrage selon la revendication 1, dans laquelle l'au moins un montant commissural flexible (328) comporte un fil unique ayant au moins un pic et un creux.

3. Structure d'ancrage selon la revendication 2, dans laquelle le fil unique a une pluralité de pics et de creux formant une pluralité d'ondes généralement sinusoïdales.

4. Structure d'ancrage selon la revendication 3 dans laquelle la fréquence et/ou la longueur d'onde des ondes sinusoïdales varient le long de l'axe longitudinal du montant commissural flexible (328).

5. Structure d'ancrage selon la revendication 3 dans laquelle la longueur d'onde des ondes sinusoïdales est sensiblement constante, le long de l'axe longitudinal du montant commissural flexible (328).

6. Structure d'ancrage selon la revendication 3 dans laquelle la flexibilité de flexion du montant commissural flexible (328) augmente à mesure que la fréquence des ondes sinusoïdales augmente et/ou la flexibilité de flexion du montant commissural flexible (328) augmente à mesure que la hauteur d'onde des ondes sinusoïdales augmente et/ou la flexibilité de flexion du montant commissural flexible (328) augmente à mesure que la distance de pic à pic entre des ondes adjacentes diminue.

7. Structure d'ancrage selon la revendication 2 dans laquelle l'anneau de flux entrant (314) comporte un premier fil de forme sinusoïdale (316) et un second fil de forme sinusoïdale (318), les premier et second fils de forme sinusoïdale (316, 318) ayant chacun une pluralité de pics (320) et de creux (322).

8. Structure d'ancrage selon la revendication 7 dans laquelle les pics du premier fil de forme sinusoïdale (316) sont joints aux creux du second fil de forme sinusoïdale (318).

9. Structure d'ancrage selon la revendication 8 dans laquelle l'au moins un montant commissural flexible comporte un élément de liaison vertical, l'élément de liaison vertical étant relié à l'anneau de flux entrant au niveau de l'intersection entre l'un des pics du premier fil de forme sinusoïdale et l'un des creux du second fil de forme sinusoïdale.

10. Structure d'ancrage selon la revendication 1 dans laquelle l'au moins un montant commissural flexible (328) comporte des premier et second fils (336, 338), chacun des fils comportant au moins un pic (340) et un creux (342).

11. Structure d'ancrage selon la revendication 10 dans laquelle l'au moins un pic (340) du premier fil (336) vient en butée contre l'au moins un creux (342) du second fil (338).

12. Structure d'ancrage selon la revendication 11 dans laquelle les premier et second fils (336, 338) comportent chacun une pluralité de pics (340) et de creux (342) formant une pluralité d'ondes généralement sinusoïdales.

13. Structure d'ancrage selon la revendication 12 dans laquelle la flexibilité de flexion du montant commissural flexible (328) augmente à mesure que la fréquence des ondes sinusoïdales des premier et second fils (336, 338) augmente et/ou la flexibilité de flexion du montant commissural flexible (328) augmente à mesure que la hauteur d'onde des ondes sinusoïdales des premier et second fils (336, 338) augmente et/ou la flexibilité de flexion du montant commissural flexible (328) augmente à mesure que la distance de pic à pic entre des ondes adjacentes des premier et second fils (336, 338) diminue.

14. Structure d'ancrage selon la revendication 1 dans laquelle la flexibilité de flexion du montant commissural (328) est supérieure à 10 % d'un montant commissural rigide ou semi-rigide.

15. Structure d'ancrage selon la revendication 1 dans laquelle la partie palette (330) de l'au moins un montant commissural flexible (328) est reliée à l'un des creux (312) ou à l'un des pics (312) de l'anneau de flux sortant (306).

16. Structure d'ancrage selon la revendication 1 comprenant en outre une valvule cardiaque bioprothétique (5) disposée à l'intérieur de la structure d'ancrage cylindrique, la valvule cardiaque bioprothétique comportant une pluralité de feuillets définissant un corps de valvule ayant une extrémité de flux entrant et une extrémité de flux sortant.

17. Structure d'ancrage selon la revendication 16 dans laquelle des feuillets adjacents sont joints ensemble par une couture (8) au niveau de la jonction des feuillets, et dans lequel une languette commissurale (9) s'étend à partir de chacune des coutures (8) près de l'extrémité de flux sortant (7) du corps de valvule.

18. Structure d'ancrage selon la revendication 17 dans laquelle la structure d'ancrage cylindrique comporte une pluralité de montants commissuraux flexibles (328), et dans laquelle la valvule cardiaque bioprothétique (5) est fixée à la structure d'ancrage cylindrique en enfilant les languettes commissurales (9) à travers les fentes axiales (332) dans les parties palette (330) des montants commissuraux flexibles (328).

19. Structure d'ancrage selon la revendication 18 dans laquelle les languettes commissurales (9) sont formées d'un seul tenant avec les feuillets.

20. Structure d'ancrage selon la revendication 18 comprenant en outre au moins un trou de suture (334) formé dans chacune des parties palette (330) pour suturer les languettes commissurales (9) aux parties palette (330).

21. Structure d'ancrage selon la revendication 18 comprenant en outre un revêtement de tissu biocompatible (550) positionné autour d'au moins une partie de l'anneau de flux entrant (514).
